Europäisches Patentamt

European Patent Office    (11) Veröffentlichungsnummer: **0 019 167**
                                                          B1
Office européen des brevets

(19)

(12) # EUROPÄISCHE PATENTSCHRIFT

(45) Veröffentlichungstag der Patentschrift:    (51) Int. Cl.³: **A 61 K  39/00**, A 61 K  35/12,
12.10.83                                              C 12 N  5/00

(21) Anmeldenummer: 80102400.1

(22) Anmeldetag: 02.05.80

(54) **Arzneipräparat zur Behandlung von Carcinomen und Verfahren zu dessen Herstellung.**

(30) Priorität: 10.05.79 DE 2918927

(43) Veröffentlichungstag der Anmeldung:
26.11.80 Patentblatt 80/24

(45) Bekanntmachung des Hinweises auf die Patenterteilung:
12.10.83 Patentblatt 83/41

(84) Benannte Vertragsstaaten:
BE DE LU NL SE

(56) Entgegenhaltungen:
DE-A-2 024 458
DE-A-2 643 215
FR-A-2 414 919

NATURE, Band 209, 12. Februar 1966 BASINGSTOKE
(GB) C.A. APFFEL et al.: "Induction of tumour immunity with tumour cells treated with iodoacetate" Seiten
694–696

NATURE, Band 248, 19. April 1974 BASINGSTOKE (GB)
C..J. SANDERSON et al.: "The induction of tumour
immunity using glutaraldehyde treated tumour cells"
Seiten 690–691

(73) Patentinhaber: Limburg, Hans, Dr., Ringstrasse 114,
D-6650 Homburg/Saar (DE)

(72) Erfinder: Limburg, Hans, Dr., Ringstrasse 114,
D-6650 Homburg/Saar (DE)

(74) Vertreter: Lehmann, Klaus, Dipl.-Ing. et al,
Patentanwälte Schroeter & Lehmann
Postfach 70 17 47 Lipowskystrasse 10,
D-8000 München 70 (DE)

Arzneipräparat zur Behandlung von Carcinomen und Verfahren zu dessen Herstellung

Die Behandlung von Carcinomen mit Cytostatica ist seit längerer Zeit bekannt. Diese haben jedoch den Nachteil, dass sie durchwegs sehr stark toxisch wirken und in der Regel auch das Wachstum von gesunden Zellen beeinträchtigen. Ausserdem hat sich herausgestellt, dass die bekannten Cytostatica nicht für alle Carcinomarten gleichermassen wirksam sind. Es kommt sogar häufig vor, dass verschiedene Patienten, die von gleichen Carcinomen befallen sind, unterschiedlich auf ein bestimmtes Cytostaticum reagieren. Um in derartigen Fällen das jeweils bestmögliche Cytostaticum zu ermitteln und selektiv eine Behandlung mit diesem durchzuführen, haben Limburg und Krahe vorgeschlagen, einen Sensibilitätstest an Gewebekulturen, die aus dem jeweiligen Carcinom gewonnen worden sind, gegenüber verschiedenen Cytostatica durchzuführen; vgl. Deutsche Medizinische Wochenschrift, Bd. 89 (1964), S. 1938.

Diese Verfahrensweise bringt zwar den Vorteil mit sich, dass der Organismus des Patienten nicht der toxischen Wirkung von Cytostatica ausgesetzt wird, die für den speziellen Tumor unwirksam sind. Jedoch bleibt immer noch die Belastung mit einem Cytostaticum, was sich insbesondere bei einer längeren Behandlungsdauer in hohen Dosen als nachteilig erweist.

Gemäss Nature, Bd. 209 (1966), S. 694–696 werden Mäuse mit experimentellen Tumoren, die mit Jodacetat vorbehandelt sind, geimpft. Durch diese Impfung soll bei den Mäusen eine Immunisierung gegen den entsprechenden Tumor entstehen.

Ein ähnlicher Sachverhalt wie bei der vorgenannten Druckschrift liegt Nature, Bd. 248 (1974), S. 690–691 zugrunde. Hier werden die Tumorzellen mit Glutaraldehyd vorbehandelt.

DE-A-2 024 458 beschreibt ein Präparat zur Immunisierung gegen Krebs durch Vorbehandlung von Tumorzellen mittels Bestrahlung.

Bisher ist nicht bekanntgeworden, dass eines dieser bekannten Präparate bei klinischen Untersuchungen eine positive Wirkung ergeben hat.

Aufgabe der Erfindung ist es, ein Arzneipräparat zur Behandlung von Carcinomen zur Verfügung zu stellen, das sich entweder zur alleinigen Behandlung oder zur gemeinsamen Behandlung mit einem speziell für den Tumor wirksamen Cytostaticum eignet. Dabei soll eine einwandfreie Verträglichkeit des Arzneipräparats durch den Patienten gegeben sein.

Diese Aufgabe wird durch das erfindungsgemässe Arzneipräparat zur Behandlung von Carcinomen gelöst, das durch einen Gehalt an Carcinomzellen gekennzeichnet ist, die mit einem Cytostaticum, gegenüber dem die Carcinomzellen empfindlich sind, so lange inkubiert worden sind, bis bei Kontrolle unter dem Mikroskop nur mehr 30 bis 70 Prozent der Zellen intakt erscheinen, wobei das Cytostaticum aus der Gruppe Cyclophosphamid, Triäthylenthiophosphoramid, Podophyllinsäure-2-äthylhydrazid, Teniposid, Vincristin, Actinomycin D, Adriblastin, 5-Fluoruracil und Methotrexat ausgewählt ist.

Vorzugsweise liegen die vorbehandelten Zellen in suspendierter Form vor.

Gegenstand der Erfindung ist ferner ein Verfahren zur Herstellung eines erfindungsgemässen Arzneipräparats, das dadurch gekennzeichnet ist, dass man

I. a) aus Carcinomzellen durch Züchtung in einem Nährmedium eine Zellkultur herstellt und
b) Proben der Zellkultur auf ihre Empfindlichkeit gegenüber Cytostatica aus der Gruppe Cyclophosphamid, Triäthylenthiophosphoramid, Podophyllinsäure-2-äthylhydrazid, Teniposid, Vincristin, Actinomycin D, Adriblastin, 5-Fluoruracil und Methotrexat testet,

II. a) unbehandelte, dem gleichen Tumor entstammende Carcinomzellen mit einem Cytostaticum, gegenüber dem beim Test gemäss I.b) Empfindlichkeit festgestellt worden ist, zur Abschwächung so lange inkubiert, bis bei Kontrolle unter dem Mikroskop nur mehr 30 bis 70 Prozent der Zellen intakt erscheinen,
b) anschliessend die abgeschwächten Zellen zur Entfernung des Cytostaticums gründlich auswäscht und
c) gegebenenfalls die abgeschwächten, ausgewaschenen Zellen in einem physiologisch verträglichen Medium suspendiert.

Vorzugsweise führt man die Inkubation gemäss Stufe II.a) 4 bis 15 Stunden, insbesondere 6 bis 12 Stunden, durch. Die Inkubationstemperatur in Stufe II.a) beträgt vorzugsweise 37°C.

Limburg und Mitarb. (Naturwissenschaften, Bd. 65 (1978), S. 394 haben zwar angeregt, eine cytostatische Krebstherapie durch Injektion von Ultrafiltraten körperspezifischer unbehandelter oder cytostatisch vorbehandelter Krebszellen zu unterstützen. Hieraus ergibt sich jedoch kein Hinweis auf die Verwendung abgeschwächter, ganzer Krebszellen.

Die Gewinnung von Carcinomzellen, die für das erfindungsgemässe Verfahren als Ausgangsmaterial verwendet werden, erfolgt auf an sich übliche Weise. Beispielsweise werden die Krebszellen aus operativ entferntem Tumorgewebe durch ein sogenanntes Trypsinierungsverfahren herausgelöst; vgl. Limburg und Mitarb., Deutsche Medizinische Wochenschrift, Bd. 89 (1964), S. 1938. Nachstehend wird eine bevorzugte Verfahrensweise bei der Gewinnung von Carcinomzellen erläutert.

Das Verfahren beginnt mit der sterilen, chirurgischen Entnahme jedes beliebigen Tumorgewebes von bösartigem Charakter während einer Operation. Die Kultivierung erfolgt nach dem Trypsinierungsverfahren auf Glimmerplättchen in LEIGHTON-tubes. Zunächst wird das entnommene Gewebe mechanisch steril zu einem Gewebsbrei zerkleinert, wobei darauf zu achten ist, dass nur festes, in der Struktur erhaltenes Krebsgewebe ohne wesentliche Blutbeimengung oder Nekrosen zur Anwendung kommt. Mit Hilfe eines Magnetrührers wird dieser Brei nach Zusatz von 0,25prozentiger Trypsinlösung (gelöst in phosphatgepufferter physiologischer Kochsalzlösung (PBS), die aus Sterilisierungsgründen 100 E/ml Penicillin und 0,1 mg/ml Streptomycin enthält) bei 37 °C gerührt. Bei manchen festeren Gewebeteilen von härterer Konsistenz kann die Trypsinlösung auch höher bis maximal 2 Prozent konzentriert werden. Nach ca. 20 Minuten werden die gelösten Einzelzellen möglichst ohne Gewebsanteile in einen zweiten, sterilen Kolben gegossen. Um die weitere Trypsinierung der abgegossenen Zellsuspension zu unterbrechen, kühlt man die Suspension mit Hilfe eines Eisbades auf + 4 °C ab. Dieser Vorgang wiederholt sich so lange, bis kein solides Gewebe mehr im Trypsinierungskolben vorhanden ist. Die gekühlte Zellsuspension wird nach Abschluss der Trypsinierung grob filtriert, am besten durch eine Mullage (die steril, aber nicht durch Chemikalien behandelt sein soll) und anschliessend bei 800 bis 1000 Umdrehungen pro Minute zentrifugiert.

Bei Verwendung von Carcinomen, die bereits in Form von Einzelzellen vorliegen, beispielsweise Ascitescarcinom- und Leukämiezellen, kann die Trypsinierungsbehandlung entfallen.

Die Züchtung der Krebszellen unter Bildung einer Zellkultur bzw. eines Zellrasens erfolgt in an sich bekannter Weise; vgl. z.B. Limburg und Mitarb., Deutsche Medizinische Wochenschau, a.a.O. Beispielsweise wird die Züchtung folgendermassen durchgeführt.

Das Zellsediment wird mit einer Nährflüssigkeit bis zu einer Zellzahl von 2 bis 4 Millionen lebender Zellen pro ml verdünnt und auf ca. 30 Röhrchen nach gutem Durchpipettieren verteilt. Ein Tropfen wird auf einen Objektträger gegeben, um die Verdünnung zu beurteilen. Ca. 20 Zellen pro Gesichtsfeld bei mittlerer (ca. 125facher) Vergrösserung entsprechen der erforderlichen Menge. Für die Untersuchung im Röhrchentest wird 1 ml Flüssigkeit pro LEIGHTON-Tube abgefüllt. Die Nährflüssigkeit setzt sich vorzugsweise wie folgt zusammen:

|  | Volumprozent |
| --- | --- |
| Gewebekulturmedium gemäss TCM Morgan, Morton, Parker (DIFCO) | 25,0 |
| Hanks-solution (+ Zugabe von 1% Phenolrot −0,5 g + NaOH n/20 bis 20 ml) | 55,0 |
| Vitamine MEM-solution (10%) | 5,0 |
| L-Glutamin (10%ig) | 5,0 |
| Kalbsserum | 10,0 |

Zur Erhaltung der Sterilität wird das Medium mit 100 E Penicillin/ml + 0,1 mg/ml Streptomycin versetzt.

Die Bebrütung der Zellsuspension geschieht im Brutschrank bei 37 °C. Der Stoffwechsel der Zellen zeigt sich durch Farbveränderung des Mediums von rot zu gelb. Bei erfolgtem Stoffwechsel wird das Medium laufend erneuert. Die Prüfung des Wachstums der Kulturen erfolgt im Auflicht (im Chemikermikroskop).

Das Wachstum der Kulturen erfolgt unterschiedlich je nach dem Ausgangsmaterial. Während ein Endometrium- oder Ovarialkarzinom bereits nach 48 Stunden zu einem guten Zellrasen ausgewachsen sein kann, benötigen andere Malignome (z.B. Plattenepithelkarzinome) je nach Reifegrad bis zu 120 Stunden.

Der Empfindlichkeitstest der Zellkulturen gegenüber verschiedenen Cytostatica erfolgt ebenfalls auf an sich bekannter Weise; vgl. Limburg und Mitarb., Deutsche Medizinische Wochenschrift, a.a.O. Beispielsweise wird hier folgendermassen vorgegangen.

Zur Testung werden nur gut ausgewachsene Zellkulturen verwendet. Prinzipiell ist die Testung je nach Materialmenge gegen jedes Cytostaticum, d.h. gleichzeitig gegen 10 bis 20 verschiedene Chemotherapeutika möglich, die in gewichtsadäquater Konzentration den Röhrchen zugegeben werden. Die Konzentration des verwendeten Mittels entspricht der therapieadäquaten Konzentration beim Menschen als Dauer- oder Stosstherapie (vgl. die in Tabelle I angegebenen Werte). Die aufgearbeiteten Medikamente werden in die Teströhrchen verteilt. Unter laufender Kontrolle im Chemikermikroskop lässt sich zumeist die morphologische Schädigung bereits nach 24 Stunden, bei Antimetaboliten nach 48 Stunden erkennen.

Folgende Konzentrationen werden im in vitro-Test therapieadäquat verwendet. Diese Dosen errechnen sich aus den von den Herstellern angegebenen Therapiekonzentrationen beim Menschen, bezogen auf ein durchschnittliches Körpergewicht von 60 kg oder auf m² Körperoberfläche und eine Blutmenge von ca. 5000 ml.

Tabelle I

| Cytostaticum | Konzentration ($\mu$g/ml) |
| --- | --- |
| Cyclophosphamid (Endoxan) | 33 |
| Triäthylenthiophosphoramid (Thio-TEPA) | 0,42 |
| Podophyllinsäure-2-äthylhydrazid (Proresid®, Mitopodozid) | 33 |
| 4′-Desmethylepipodophyllotoxin-β-D-4,6-thenylidenglucosid (VM 26, Teniposid) | 1 |
| Vincristin | 0,017 |
| Actinomycin D | 0,05 |
| Adriblastin | 1 |
| 5-Fluoruracil | 16,7 |
| Methotrexat (A-Methopterin) | 2 |

Die Sensibilität bzw. Resistenz einer menschlichen Krebsgewebekultur wird semiquantitativ ermittelt. Bei völliger Resistenz gleicht die Kultur völlig derjenigen des Kontrollröhrchens. Eine Teilwirkung erkennt man an einer Teilauflösung der Zellen pro Gesichtsfeld von ca. 50 Prozent. Bei hoher Sensibilität besteht nur noch ein zellfreies Blickfeld mit wenigen geschrumpften (abgetöteten) Einzelzellen. Nur diese Wirkung gilt als voll sensibles Ergebnis. Im Durchschnitt sind mindestens 2 mit verschiedenen Cytostatica behandelte Kulturen sensibel. Nur diese werden später für die noch zu erläuternde Zelltherapie verwendet. Das Besondere an diesen Testergebnissen, die bisher in über 3000 Einzelfällen durchgeführt worden sind, ist die völlig unterschiedliche individuelle Reaktion jedes einzelnen Tumors trotz gleicher Herkunft bzw. Histologie auf das gleiche Cytostaticum bei verschiedenen Tumorträgern. Auch die Metastasen können different vom Primärtumor reagieren.

Zum Testen der Carcinomkulturen auf ihre Empfindlichkeit gegenüber verschiedenen Cytostatica kann auch ein in vivo-Verfahren im Rattenabdomen durchgeführt werden.

Die in vivo-Technik mit Diffusionskammern im Rattenabdomen wird angewendet, wenn weniger als 20 g Tumormaterial zur Verfügung steht. Die Aufarbeitung der Zellsuspension ist dabei die gleiche wie im in vitro-Verfahren. Die Diffusionskammer besteht aus 2 Filtern, die in 2 aneinanderschraubbare Teflonringe gelegt werden. Die Filter wirken als semipermeable Membran. Die Porengrösse ist so gestaltet, dass zwar Flüssigkeit, Elektrolyte und Proteine die Membran passieren können, jedoch ein Durchwandern von Zellen (Leukozyten, Makrophagen) unmöglich ist. Die Porengrösse der Filter beträgt 0,25 Mikrometer. Die so gebildete Kammer wird mit 0,3 ml der präparierten Zellsuspension gefüllt. Die gefüllten Kammern werden unmittelbar nach der Aufarbeitung in die Peritonealhöhle von Inzuchtratten des Stammes BD 2 implantiert. Pro Tier werden zwei Kammern verwendet. Nach Implantation werden Peritoneum und Bauchdecken verschlossen. Bei dieser Methode wird eine Angehrate der Tumoren auf den Membranfiltern von 100 Prozent erreicht. Am 5. Tag nach der Implantation der Kammern in das Rattenebdomen erfolgt die Injektion der verschiedenen Cytostatica in die Peritonealhöhle der Ratten. Die Konzentration der Mittel entspricht ebenso wie beim in vitro-Verfahren einer Therapie beim Menschen. 48 Stunden nach der letzten Injektion werden die Ratten getötet und die Diffusionskammern entfernt. Die Membranfilter werden isoliert, fixiert und die Zellen unmittelbar auf den Filtern nach Papanicolaou gefärbt. Die Beurteilung des cytotoxischen Effekts erfolgt nach denselben morphologischen Kriterien wie beim in vitro-Verfahren.

Anschliessend an die vorstehend erläuterte Austestung der Zellen in bezug auf ihre Sensibilität gegenüber verschiedenen Cytostatica erfolgt die erfindungsgemässe Abschwächungsbehandlung von unbehandelten Carcinomzellen der gleichen Herkunft wie die vorher ausgetesteten Zellen.

Wesentlich für das erfindungsgemässe Verfahren ist, dass nach Ermittlung des optimalen Cytostaticums für die jeweilige Zellkultur die Zellen nicht vollständig abgetötet werden, sondern nur eine teilweise Abschwächung vorgenommen wird. Hierzu muss sorgfältig darauf geachtet werden, dass die Inkubationsdauer mit dem Cytostaticum nicht zu lange ist. Die jeweilige Inkubationszeit hängt von den einzelnen Zellkulturen sowie von der Wirksamkeit des Cytostaticums ab. Der Abschwächungsvorgang lässt sich unter dem Mikroskop bei etwa 125facher Vergrösserung beobachten. Vorzugsweise wird so lange inkubiert, bis bei der mikroskopischen Kontrolle nur mehr etwa 30 bis 70 Prozent und vorzugsweise etwa 40 bis 60 Prozent der Zellen als intakt erscheinen. Im allgemeinen haben sich Inkubationszeiten von etwa 4 bis 15 Stunden und insbesondere von 6 bis 12 Stunden als brauchbar erwiesen. Die Inkubationstemperatur beträgt vorzugsweise etwa 37 °C. Die Cytostaticakonzentrationen entsprechen bei dieser Abschwächungsbehandlung den Konzentrationen, die bei der vorerwähnten Austestung angewendet werden.

Vorzugsweise wird die Abschwächungsbehandlung folgendermassen durchgeführt: Die unbehandelte, nach dem vorstehenden Verfahren durch Trypsinieren erhaltene Suspension von Zellen im Nährmedium wird in Roux-Flaschen gegeben und im Brutschrank auf 37 °C erwärmt. In die Flaschen wird das Cytostaticum gegeben, das sich beim vorstehenden Test als optimal erwiesen hat. Die Mengen entsprechen dabei den in Tabelle I angegebenen Konzentrationen.

Anschliessend an die Abschwächung der Zellen ist dafür zu sorgen, dass das Cytostaticum gründlich ausgewaschen wird. Dies dient vor allem dazu, um eine Weiterreaktion des Cytostaticums mit den Zellen unter möglicher Abtötung der Zellen zu verhindern. Zum Waschen wird vorzugsweise PBS verwendet.

Zweckmässigerweise werden die auf diese Weise abgeschwächten Zellen in einem physiologisch verträglichen Medium, wie physiologische Kochsalzlösung oder Kälberserum, suspendiert. Die Zellkonzentrationen in derartigen verabreichungsfertigen Präparaten betragen vorzugsweise $10^6$ Zellen pro ml.

Vorzugsweise werden die erfindungsgemässen Präparate aus körpereigenen Tumorzellen des zu behandelnden Patienten hergestellt. Dies gewährleistet eine besonders gute Verträglichkeit sowie eine optimale Wirkungsweise. In Fällen, wo eine Herstellung des Präparats aus körpereigenen Zellen aus Zeitgründen oder wegen mangelnder Ausrüstung und Praxis in der Herstellung und Untersuchung von Carcinomgewebekulturen nicht möglich ist, können auch fremde Carcinomzellpräparate verabreicht werden. Hierbei wird vorzugsweise darauf geachtet, dass jeweils die entsprechenden Zellpräparate gegeben werden, z.B. aus Ovarialcarcinomen gewonnene Präparate bei Patientinnen mit Ovarialcarcinom und dergleichen. Möglich ist auch die Herstellung von Mischpräparaten, die aus verschiedenen Carcinomarten gewonnene Zellen enthalten, z.B. eine Kombination aus abgeschwächten Mamma- und Ovarialcarcinomzellen.

Die erfindungsgemässen Präparate erweisen sich in suspendierter Form ca. 4 Wochen lang bei einer Lagertemperatur von +4 °C stabil. Um eine längere Lagerfähigkeit der Präparate zu erreichen, werden sie zweckmässigerweise rasch auf Temperaturen bis −80 °C eingefroren. In tiefgefrorenem Zustand sind die Präparate ca. 1 Jahr stabil. Hierzu werden sie vorzugsweise mit einem geringen Überschuss Nährlösung eingeschmolzen. Die Präparate können auch in lyophilisiertem Zustand längere Zeit gelagert werden.

Die erfindungsgemässen Präparate können subkutan, intramuskulär oder langsam intravenös verabfolgt werden. Die subkutane oder intramuskuläre Injektion wird bevorzugt. Vorzugsweise werden etwa 10 ml Zellsuspension mit einer Zellmenge von $10^6$ Zellen bei einer Behandlung gegeben. Die Behandlung wird zweckmässigerweise 5- oder 6mal in Abständen von etwa 48 Stunden wiederholt. Es hat sich herausgestellt, dass nach einer Verabfolgung der erfindungsgemässen Präparate eine erheblich verbesserte Verträglichkeit der entsprechenden chemischen Cytostatica gegeben ist. Somit ist es besonders zweckmässig, ca. 8 Tage nach einer Behandlung mit den erfindungsgemässen Präparaten eine herkömmliche Behandlung mit Cytostatica durchzuführen. Nähere Einzelheiten hierzu ergeben sich aus dem nachstehenden klinischen Bericht.

Beispiel

Es werden folgende Reagentien und Lösungen verwendet:

Waschlösung:
80 ml Lösung A,
100 ml Lösung B,
100 ml Lösung C und
5 ml Antibiotikalösung
mit bidestilliertem Wasser auf 1000 ml auffüllen.

Lösung A:

| | | |
|---|---|---|
| NaCl | 40,0 | g |
| KCl | 1,0 | g |
| $Na_2HPO_4 \times 2H_2O$ | 5,75 | g |
| $KH_2PO_4$ | 1,0 | g |

in 400 ml bidestilliertem Wasser lösen.

Lösung B:

| | |
|---|---|
| $CaCl_2$ | 0,5 g |

in 500 ml bidestilliertem Wasser lösen.

Lösung C:

| | |
|---|---|
| $MgCl_2 \times 6H_2O$ | 0,5 g |

in 500 ml bidestilliertem Wasser lösen.

Die vorgenannten Lösungen werden im Autoklav sterilisiert und bei 4 °C aufbewahrt.

Antibiotikalösung:
Penicillin (Grünenthal, Stolberg) 6 Millionen i.E.
Streptomycin (Specia, Paris) 1 g
in 300 ml bidestilliertem Wasser lösen und bei −20 °C aufbewahren.

Trypsinlösung:

| | |
|---|---|
| Trypsinpulver (Serva, Heidelberg) | 25 g |
| Hanks(BSS)-Lösung (vgl. unten) | 475 ml |

Die Lösung wird bei 37 °C im Wasserbad hergestellt, steril filtriert, in kleine Flaschen abgefüllt und bei −20 °C aufbewahrt. Für die Trypsinierungsbehandlung wird diese Lösung mit Waschlösung auf die gewünschte Konzentration eingestellt.

Nährmedium Behring IV:

| | |
|---|---|
| Hanks-Lösung (55 ml Stammlösung A + 55 ml Stammlösung B mit bidestilliertem Wasser auf 1100 ml aufgefüllt) | 1100 ml |
| TCM-Lösung | 500 ml |
| Kälberserum | 200 ml |
| Vitaminlösung | 100 ml |
| Glutaminlösung | 100 ml |
| Antibiotikalösung | 10 ml |

Das Gemisch wird nach steriler Filtration in Flaschen abgefüllt und bei −20 °C aufbewahrt. Vor Gebrauch wird das Medium mit Natriumhydrogencarbonatlösung (22 g $NaHCO_3$ und 478 ml bidestilliertes Wasser) auf den pH-Bereich 6,8 bis 7,2 eingestellt.

Hanks-Stammlösung A:

| | |
|---|---|
| NaCl | 40,0 g |
| KCl | 2,0 g |
| $MgSO_4 \times 7H_2O$ | 0,5 g |
| $MgCl_2 \times 6H_2O$ | 0,5 g |
| $CaCl_2$ | 0,7 g |

In 250 ml bidestilliertem Wasser lösen und bei 4 °C aufbewahren.

Hanks-Stammlösung B:

| | |
|---|---|
| $Na_2HPO_4 \times 2H_2O$ | 0,3 g |
| $KH_2PO_4$ | 0,3 g |
| Glucose | 5,0 g |
| Phenolrotlösung 1%ig | 10,0 ml |

In 240 ml bidestilliertem Wasser lösen und bei 4 °C aufbewahren.

Phenolrotlösung:

| | |
|---|---|
| Phenolrot | 0,5 g |
| 1/20 n NaOH | 15 ml |

Das Phenolrot wird innerhalb von etwa 30 Minuten in der Kälte in der Natronlauge gelöst und sodann mit bidestilliertem Wasser auf ein Volumen von 50 ml aufgefüllt. Die Lösung wird bei 4 °C aufbewahrt.

TCM-Lösung:

| | |
|---|---|
| TCM-Pulver (gemäss Morgan, Morton und Parker; Difco Laboratories, Detroit, USA) | 5,5 g |
| Hanks A-Stammlösung | 25 ml |
| Hanks B-Stammlösung | 25 ml |

Die beiden Stammlösungen werden mit bidestilliertem Wasser auf 500 ml aufgefüllt. In der erhaltenen Lösung wird das Pulver gelöst.

Glutaminlösung:

| | |
|---|---|
| Glutaminpulver (Difco Laboratories) | 10,0 g |
| Hanks A-Stammlösung | 6 ml |
| Hanks B-Stammlösung | 6 ml |

Die Stammlösungen werden mit bidestilliertem Wasser auf ein Volumen von 100 ml aufgefüllt. In dieser Lösung wird das Glutaminpulver gelöst.

Vitaminlösung:

Vitamin (MEM 100 x, Boehringer, Mannheim)
                 100 ml
Hanks A-Stammlösung        50 ml
Hanks B-Stammlösung        50 ml

Die Stammlösungen werden mit bidestilliertem Wasser auf ein Volumen von 900 ml aufgefüllt. Die Vitamine werden zugesetzt. Sodann wird steril filtriert, in kleine Flaschen von 100 ml abgefüllt und bei −20 °C aufbewahrt.

Kälberserum:

Fötales Kälberserum (Boehringer, Mannheim) wird bei −20 °C aufbewahrt und vor dem Verbrauch im Wasserbad bei 37 °C aufgetaut.

50 g Gewebe, entnommen aus einem Ovarialcarcinom einer 46jährigen Patientin im Stadium T4N5M1c, werden manuell zerkleinert und zusammen mit 700 ml verdünnter Trypsinlösung (0,5prozentig) in einen 1000 ml fassenden Kolben gegeben. Das Gemisch wird 10 Minuten mit Hilfe eines Magnetrührers gerührt. Anschliessend wird der Überstand in einen eisgekühlten Kolben gegossen. Dieser Vorgang wird insgesamt 4mal durchgeführt. Beim letztenmal ist der Überstand klar. Der vereinigte Überstand und das verbliebene Gewebe werden sodann durch sterilen Mull filtriert. Das Filtrat wird 10 Minuten bei 800 bis 1000 U./min zentrifugiert. Das Sediment wird mit 100 ml Waschlösung aufgegossen und erneut zentrifugiert.

Das erhaltene Sediment wird mit 5 ml Nährmedium gut durchpipettiert. Sodann wird mit Nährmedium auf ein Gesamtvolumen von ca. 400 ml aufgefüllt. Bei dieser Verdünnung finden sich bei Betrachtung unter einem Zeiss-Umkehrmikroskop (125fache Vergrösserung) ca. 20 bis 25 Zellen im Blickfeld. Die verdünnte Zellsuspension wird auf Reagensröhrchen (1 ml pro Röhrchen) und auf Roux-Flaschen (100 ml pro Flasche) verteilt. 6 Tage lang wird das Zellwachstum und der Stoffwechsel täglich beurteilt. Sobald das Medium einen Farbumschlag von rot nach gelb erkennen lässt, wird die Nährlösung erneuert. Nach 6tägiger Bebrütung der Zellsuspension bei 37 °C im Brutschrank ergibt sich ein gutes Zellwachstum.

Mit den in den Röhrchen befindlichen Zellkulturen wird der vorstehend erläuterte in vitro-Test mit den in Tabelle I aufgeführten Cytostatica in den dort angegebenen Konzentrationen durchgeführt. Die Beurteilung der morphologischen Schädigung unter dem Mikroskop wird nach 24 Stunden durchgeführt. Hierbei erweist sich die Zellsuspension gegenüber folgenden Cytostatica als empfindlich: Adriblastin, Vincristin und Proresid®. Alle übrigen (in diesem Fall 6) Cytostatica waren unwirksam.

Sodann werden 4 Roux-Flaschen mit der vorstehend erläuterten Zellsuspension bereitgestellt. Je 1 Flasche wird mit Adriblastin, Vincristin und Proresid in den in Tabelle I angegebenen Konzentrationen behandelt. Die Zellsuspension in der restlichen Flasche bleibt unbehandelt und dient als Kontrolle. Nach 12stündiger Einwirkung erweisen sich bei Kontrolle unter dem Mikroskop nur mehr 60 Prozent der Zellen als intakt. Hierauf werden die Zellen durch 10minutige Zentrifugation bei 800 bis 1000 U./min abzentrifugiert. Das Sediment wird mit 10 ml frischem Nährmedium aufgegossen. Die erhaltenen, abgeschwächten Zellen werden in dem frischen Nährmedium in einer Zellkonzentration von $10^6$ Zellen/ml suspendiert. Die Suspension wird bei 4 °C aufbewahrt.

In vitro-Versuch

In Reagensröhrchen werden jeweils 1 ml einer unbehandelten Zellsuspension mit $10^6$ Zellen gegeben. Bei diesen Zellen handelt es sich um die unbehandelten Zellen, die gemäss vorstehendem Beispiel durch Trypsinierung des Carcinomgewebes erhalten worden sind. In jedes Röhrchen werden 6 Tage lang täglich 0,3 ml der vorstehenden drei Präparate (mit Adriblastin, Vincristin bzw. Proresid abgeschwächte Zellen) gegeben. Hierbei befinden sich die Röhrchen bei einer Temperatur von 37 °C im Brutschrank. Nach 7 bis 9 Tagen sind die unbehandelten Zellen im Röhrchen zu etwa 80 bis 90 Prozent durch die erfindungsgemässen Präparate zerstört. Da die Cytostatica aus den Präparaten gründlich ausgewaschen sind, handelt es sich hierbei um eine Zellreaktion.

Klinische Behandlung

Aufgrund des vorstehenden Versuchs und weiterer ähnlicher Versuche sowie nach entsprechenden Versuchen an Tiertumorstämmen wurde eine klinische Anwendung der erfindungsgemässen Präparate vorgenommen. Mit einer Ausnahme litten alle Patientinnen an Krebserkrankungen in aussichtslosen Stadien und befanden sich trotz Operationen, Bestrahlungen und/oder cytostatischen Behandlungen in den Stadien T4 N3-5 M1c (Klassifikation nach der Union International contre le Cancer). Die durchschnittliche Überlebenszeit derartiger Patienten beträgt weniger als 60 Tage. Die erfindungsgemässen Präparate wurden hierbei hauptsächlich zur adjuvanten Immuntherapie eingesetzt, d. h. die Präparate sollten eine Verbesserung des Immunsystems bewirken und eine anschliessende cytostatische Behandlung mit den entsprechenden Cytostatica vorbereiten. Insgesamt wurden 22 Patientinnen, die über ihren Zustand und die beabsichtigte Behandlung voll aufgeklärt waren, behandelt. Es handelte sich insgesamt um 14 Ovarialmalignome (hierunter 1 Rezidiv im Mesosigma), 4 Mammacarcinome, 1 Pankreascarcinom, 1 Retothelsarkom, 1 Coecalcarcinom und 1 Carcinom des Corpus uteri.

Den Patientinnen wurde durch Operation Krebsgewebe entnommen. Hieraus wurden entsprechend den vorstehenden Angaben die jeweiligen erfindungsgemässen Präparate gewonnen. Den Patientinnen wurden 6- bis 8mal jeden zweiten Tag intraglutaeal je 10 ml Zellsuspension mit insgesamt $10^6$ Zellen (1 ml Präparat in Nährlösung und 9 ml physiologische Kochsalzlösung oder Kälberserum), gegebenenfalls zusammen mit 1 ml 0,5prozentiger Lidocainlösung (Xylocain), verabfolgt. Die Häufigkeit der Injektionen richtet sich bei der Erstbehandlung nach der Menge der zur Verfügung stehenden Zellen, d.h. zumeist zwischen 5- und 8mal/48 Std. Nach einer Wartezeit von 8 bis 20 Tagen (bei Patientinnen in schlechtem Zustand liegt die Wartezeit an der Obergrenze) erfolgte dann die cytostatische Behandlung entsprechend dem Testergebnis, zumeist nach folgendem Schema: Vincristin 1 mg intravenös, 6 Stunden später 40 mg/m² Körperoberfläche Adriblastin intravenös und 48 Stunden später 25 mg/kg Körpergewicht Cyclophosphamid oder Proresid, gelöst in der gleichen Konzentration in 500 ml physiologischer Kochsalzlösung. Mit Rücksicht auf die Toxizität von Adriblastin wurde zwischen 2 cytostatischen Behandlungen ein Abstand von 3 Wochen eingehalten, bis insgesamt 550 mg dieses Mittels verabreicht waren. Bei Resistenz wurde auf andere Cytostatica, wie 5-FU, Methotrexat-Leukoverin und dergleichen, in wöchentlichen Abständen umgestellt.

Bei dieser Behandlung ging es sämtlichen Patientinnen subjektiv und objektiv auffallend besser. Es ergaben sich keine lokalen oder allgemeinen Nebenwirkungen. Trotz der an sich zu vermutenden Toxizität trat bei der Injektion der Zellsuspension keine Rötung oder Schwellung auf. Es konnte auch keine Schmerzempfindung oder eine andere Reaktion auf die Injektion festgestellt werden. Die Besserung des Zustands war in sämtlichen Fällen so nachhaltig, dass nach einem Intervall von maximal 20 Tagen die an sich gefährliche cytostatische Therapie eingeleitet werden konnte.

4 der vorgenannten 22 Patientinnen erhielten keine zusätzliche cytostatische Therapie mehr, da sie diese ablehnten oder die Tumoren zu weit fortgeschritten erschienen. Dennoch überlebten auch diese Patientinnen aussergewöhnlich lange. Beispielsweise zeigte eine 60jährige Frau mit einem über faustgrossen Primärtumor der Brustdrüse und ossären wie auch nachgewiesenen Gehirnmetastasen, bei der lediglich eine einfache Ablatio mammae durchgeführt und anschliessend die Behandlung mit dem erfindungsgemässen Präparat durchgeführt wurde, ohne sonstige Therapie eine Überlebenszeit von 270 Tagen.

Bei 5 Patientinnen wurde auch eine Behandlung mit heterologen Carcinomzellsuspensionen durchgeführt. Auch hierbei wurde eine sehr gute Verträglichkeit beobachtet.

Die durchschnittliche Überlebenszeit sämtlicher Patientinnen beträgt zur Zeit 350 Tage, also fast das 6fache des bisherigen Durchschnitts. 5 Patientinnen sind nach 245 bis 665 Tagen noch am Leben, hiervon 3 ohne, 2 mit weiter behandlungsfähigen Rezidiven. Alle sind in gutem Allgemeinzustand.

Nachstehend wird die Wirkung der erfindungsgemässen Präparate auf 2 der vorstehend genannten 22 Patientinnen sowie auf eine weitere Patientin erläutert:

1. Eine 46jährige Patientin zeigte bei der Aufnahme doppelseitige Mammatumoren, die seit 2 Jahren bestanden. Ferner bestanden grosse Unterleibstumoren. Der Allgemeinzustand der Patientin war äusserst schlecht. Nach allgemeiner Vorbehandlung wurden ihr in 2 operativen Sitzungen im Juni 1978 zunächst beide Brüste, die einen ausgedehnten Krebsbefall aufwiesen, zusammen mit den zugehörigen Lymphknoten der Axilla entfernt. Nach 14 Tagen wurden der Uterus und die beiden Ovarialtumoren entfernt. Restliches Krebsgewebe im grossen Netz und auf dem Bauchfell, Lymphknoten und an anderen Stellen musste zurückgelassen werden. Die Patientin erhielt keine Bestrahlung, jedoch insgesamt 12 Injektionen mit dem erfindungsgemässen Präparat aus vorbehandelten, körpereigenen Carcinomzellen (Präparat gemäss dem Beispiel) und anschliessend eine kombinierte Chemotherapie. Unter der Immuntherapie kam es zur vollständigen Erholung der Patientin und guter Verträglichkeit der Cytostatica. Nach Ablauf von insgesamt 12 Monaten geht es der Patientin ausgezeichnet. Sie kann voll ihrer Arbeit nachgehen und hat keinerlei Beschwerden. Klinisch ist sie ohne jedes Anzeichen für Rezidiv, weder abdominal noch im Brustraum. Das Tumorstadium bei Behandlungsbeginn war T4 N5 M1c.

2. Eine 32jährige Patientin kam mit einem grossen Unterbauchtumor zur Aufnahme, nachdem ihr 1 Jahr früher an anderer Stelle ein grösserer Eierstockkrebs durch Entfernung des Uterus und beider Anhänge behandelt worden war. Sie hatte auch eine cytostatische Behandlung durchgemacht. Nach Relaparotomie wurde ein fast kindskopfgrosser Tumor des Mesosigma als Rezidiv des früheren Ovarialcarcinoms reseziert, der jedoch nicht völlig im Gesunden entfernt werden konnte. Ferner bestanden Lymphknotenmetastasen an der Beckenwand. Sie erhielt postoperativ gleichfalls eine adjuvante Immuntherapie aus 12 Injektionen ihrer eigenen, behandelten Tumorzellen. Eine cytostatische Therapie konnte aus technischen Gründen, da sich die Patientin im Ausland aufhielt, nicht nachvollzogen werden. Die Patientin kam aber mehrfach zur Nachuntersuchung. 11 Monate nach Beginn der Immuntherapie lebt sie in ausgezeichnetem Zustand ohne klinische Anzeichen von Rezidiv. Das gesamte Abdomen ist frei von Tumorbildungen.

3. Eine 41jährige Patientin, die wegen des vorliegenden T1-Stadiums im vorstehenden Kollektiv nicht enthalten ist, kam zur Klinikaufnahme wegen eines kleinen krebsverdächtigen Knotens in der linken Brustdrüse. Mammographie, Thermographie und Punktionscytologie waren links positiv, rechts in einem weiteren kleinen Knoten zweifelhaft (Papanicolaou 3). Die Tumorexcision links zeigte histologisch ein Carcinoma mammae scirrhosum (T1 Nx Mo) von ca. 2 cm Durchmesser. Es handelte sich um den einzigen Frühfall des Patientenkollektivs, der deshalb auch gesondert mitgeteilt wird. Die Patientin willigte in eine einfache Mastektomie der linken Brust ein, lehnte aber weitere Massnahmen, insbesondere an der rechten Brust, ab. Ferner lehnte sie eine cytostatische oder sonstige Nachbehandlung ab, war aber mit der Zellbehandlung einverstanden. Sie erhielt eine heterologe Zellbehandlung mit erfindungsgemäss behandelten Mammacarcinomzellen einer anderen Patientin nach vorstehendem Behandlungsschema (6mal 10 ml Zellsuspension mit $10^6$ Zellen jeden zweiten Tag i.m.). Die Patientin lebt 12 Monate nach der Behandlung ohne jedes Zeichen von Rezidiv. Der suspekte Tumor der rechten Mamma ist völlig verschwunden.

Aus den bisherigen Untersuchungen hat sich ergeben, dass in den meisten Fällen aufgrund von Materialmangel eine zu geringe Dosierung erfolgen musste. Wünschenswert wäre bei jedem Carcinom, auch bei Carcinomen im Anfangsstadium, eine Injektionstherapie 5- oder 6mal im Abstand von 48 Stunden monatlich, dann 3mal im Abstand von 2 Monaten, hierauf 4mal im Halbjahresabstand und schliesslich noch mehrere Jahre im Ganzjahresabstand. Hierfür könnten dann nur heterologe Krebszellen, die nach Möglichkeit aber aus den gleichen Organen stammen, dienen, falls keine autologen Zellen zur Verfügung stehen. Sinngemäss kann man den heterologen Krebszellen auch lyophilisierte Krebszellen, die keiner Vorbehandlung bedürfen, beimischen (Verhältnis 1:1).

**Patentansprüche**

1. Arzneipräparat zur Behandlung von Carcinomen, gekennzeichnet durch einen Gehalt an Carcinomzellen, die mit einem Cytostaticum, gegenüber dem die Carcinomzellen empfindlich sind, so lange inkubiert worden sind, bis bei Kontrolle unter dem Mikroskop nur mehr 30 bis 70 Prozent der Zellen intakt erscheinen, wobei das Cytostaticum aus der Gruppe Cyclophosphamid, Triäthylenthiophosphoramid, Podophyllinsäure-2-äthylhydrazid, Teniposid, Vincristin, Actinomycin D, Adriblastin, 5-Fluoruracil und Methotrexat ausgewählt ist.

2. Arzneipräparat nach Anspruch 1, dadurch gekennzeichnet, dass die vorbehandelten Zellen in suspendierter Form vorliegen.

3. Verfahren zur Herstellung eines Arzneipräparats nach Anspruch 1, dadurch gekennzeichnet, dass man
I. a) aus Carcinomzellen durch Züchtung in einem Nährmedium eine Zellkultur herstellt und
b) Proben der Zellkultur auf ihre Empfindlichkeit gegenüber Cytostatica aus der Gruppe Cyclophosphamid, Triäthylenthiophosphoramid, Podophyllinsäure-2-äthylhydrazid, Teniposid, Vincristin, Actinomycin D, Adriblastin, 5-Fluoruracil und Methotrexat testet,
II. a) unbehandelte, dem gleichen Tumor entstammende Carcinomzellen mit einem Cytostaticum, gegenüber dem beim Test gemäss I.b) Empfindlichkeit festgestellt worden ist, zur Abschwächung so lange inkubiert, bis bei Kontrolle unter dem Mikroskop nur mehr 30 bis 70 Prozent der Zellen intakt erscheinen,

b) anschliessend die abgeschwächten Zellen zur Entfernung des Cytostaticums gründlich auswäscht und
c) gegebenenfalls die abgeschwächten, ausgewaschenen Zellen in einem physiologisch verträglichen Medium suspendiert.

4. Verfahren nach Anspruch 2, dadurch gekennzeichnet, dass man die Inkubation gemäss Stufe II.a) 4 bis 15 Stunden durchführt.

5. Verfahren nach Anspruch 2, dadurch gekennzeichnet, dass man die Inkubation gemäss Stufe II.a) 6 bis 12 Stunden durchführt.

6. Verfahren nach Anspruch 2, dadurch gekennzeichnet, dass man die Inkubation gemäss Stufe II.a) bei 37 °C durchführt.

**Claims**

1. Medicinal preparation for the treatment of carcinoma, characterized in that it contains carcinoma cells which have been incubated by a cytostatic agent to which the carcinoma cells are sensitive, until only 30 to 70 percent of the cells appear to be intact under microscopic examination, the cytostatic agent being selected from the group of cyclophosphamide, triethylene-thiophosphoramide, podophyllic acid-2-ethylhydrazide, teniposide, vincristin, actinomycin D, adriblastin, 5-fluoruracil and methotrexate.

2. Medicinal preparation according to claim 1, characterized in that the pretreated cells are present in a suspension.

3. Process for preparing a medicinal preparation according to claim 1, characterized in that
I. a) carcinoma cells are cultured in a nutrient medium to form a cell culture, and
b) samples of the cell culture are tested for their sensitivity to cytostatic agents selected from the group of cyclophosphamide, triethylene-thiophosphoramide, podophyllic acid-2-ethylhydrazide, teniposide, vincristin, actinomycin D, adriblastin, 5-fluoruracil and methotrexate,
II. a) untreated carcinoma cells originating from the same tumor are weakened by being incubated with a cytostatic agent for which a sensitivity has been ascertained in the test according to I.b), until only 30 to 70 percent of the cells appear to be intact under microscopic examination,
b) thereafter, the weakened cells are thoroughly washed to remove the cytostatic agent, and
c) optionally, the weakened, washed cells are suspended in a physiologically tolerable medium.

4. Process according to claim 2, characterized in that the incubation of step II.a) is carried out for 4 to 15 hours.

5. Process according to claim 2, characterized in that the incubation of step II.a) is carried out for 6 to 12 hours.

6. Process according to claim 2, characterized in that the incubation of step II.a) is carried out at 37 °C.

## Revendications

1. Préparation pharmaceutique pour le traitement de carcinomes, caractérisée en ce qu'elle contient des cellules de carcinomes qui ont été mises en incubation avec un agent cytostatique auquel ces cellules sont sensibles, jusqu'à ce qu'à l'examen microscopique, on ait constaté que seulement 30 à 70% des cellules sont intactes, l'agent cytostatique étant choisi dans le groupe cyclophosphamide, triéthylène thiophosphoramide, acide podophyllique-2-éthylhydrazide, Teniposide, Vincristine, actinomycine D, adriblastine, 5-Fluoruracil et méthotrexate.

2. Préparation pharmaceutique suivant la revendication 1, caractérisé en ce que les cellules prétraitées sont en suspension.

3. Procédé de fabrication d'une préparation pharmaceutique suivant la revendication 1, caractérisé en ce que

I. a) on met des cellules de carcinome en culture dans un milieu nutritif et

b) on teste des échantillons de la culture de cellules en ce qui concerne leur sensibilité aux agents cytostatiques du groupe cyclophosphamide, triéthylène thiophosphoramide, acide podophyllique-2-éthylhydrazide, Teniposide, Vincristine, actinomycine D, adriblastine, 5-Fluoruracil et méthotrexate.

II. a) on incube, pour affaiblissement, des cellules de carcinomes non traitées, provenant de la même tumeur, avec un agent cytostatique, à l'égard duquel une sensibilité dans le test suivant I.b) a été constatée, cette incubation durant jusqu'à ce qu'à l'examen microscopique, on constate que seulement 30 à 70% des cellules sont intactes,

b) ensuite on lave soigneusement les cellules affaiblies, pour éliminer l'agent cytostatique, et

c) le cas échéant, on met en suspension, dans un fluide physiologiquement acceptable, les cellules affaiblies et lavées.

4. Procédé suivant la revendication 2, caractérisé en ce que l'incubation suivant l'étape II.a) dure 4 à 15 heures.

5. Procédé suivant la revendication 2, caractérisé en ce que l'incubation suivant l'étape II.a) dure 6 à 12 heures.

6. Procédé suivant la revendication 2, caractérisé en ce que l'incubation suivant l'étape II.a) est réalisée à 37 °C.